(19) Europäisches Patentamt
European Patent Office
Office européen des brevets

(11) Publication number: **0 297 816 B1**

(12) **EUROPEAN PATENT SPECIFICATION**

(45) Date of publication of patent specification: **02.03.94**

(51) Int. Cl.[5]: **C07K 5/02**, A61K 37/64

(21) Application number: **88305847.1**

(22) Date of filing: **24.06.88**

The file contains technical information submitted after the application was filed and not included in this specification

(54) **Homocyclostatine and cyclostatine containing polypeptides as antihypertensive agents.**

(30) Priority: **01.07.87 US 68992**

(43) Date of publication of application:
**04.01.89 Bulletin 89/01**

(45) Publication of the grant of the patent:
**02.03.94 Bulletin 94/09**

(84) Designated Contracting States:
**AT BE CH DE ES FR GB GR IT LI LU NL SE**

(56) References cited:
**EP-A- 0 184 855**
**EP-A- 0 212 903**
**EP-A- 0 220 665**
**EP-A- 0 314 239**

(73) Proprietor: **PFIZER INC.**
**235 East 42nd Street**
**New York, N.Y. 10017(US)**

(72) Inventor: **Hoover, Dennis Jay**
**5, Fargo Drive**
**Ledyard, CT(US)**
Inventor: **Rosati, Robert Louis**
**RD 3, Box 66a,**
**Deans Mill Road**
**Stonington, CT(US)**

(74) Representative: **Moore, James William, Dr.**
**Pfizer Limited**
**Ramsgate Road**
**Sandwich Kent CT13 9NJ (GB)**

EP 0 297 816 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel homocyclostatine containing polypeptides useful and antihypertensive agents.

The proteolytic enzyme renin, which has a molecular weight of about 40,000, is produced in and secreted into the blood by the kidney. It is known to be active in vivo in cleaving the naturally-occurring plasma glycoprotein angiotensinogen, in the case of human angiotensinogen at the bond between the leucine (10th) and valine (11th) amino acid residues at the N-terminal end of the angiotensinogen:

Asp-Arg-Val-Tyr-Ile-His-Pro-Phe-His-Leu-Val-
1 2 3 4 5 6 7 8 9 10 11
Ile-His-Ser-Glu-
12 13 14 15

The circulating N-terminal decapeptide (angiotensin I) formed by the above cleaving action of renin is subsequently broken down by the body to an octapeptide known as angiotensin II. Angiotensin II is known to be a potent pressor substance, i.e. a substance that is capable or inducing a significant increase in blood pressure and is believed to act by causing the constriction of blood vessels and the release of the sodium-retaining hormone aldosterone from the adrenal gland. Thus, the renin-angiotensinogen system has been implicated as a causative factor in certain forms of hypertension and congestive heart failure.

One means of alleviating the adverse effects of the functioning of the renin-angiotensinogen system is the administration of a substance capable of inhibiting the angiotensinogen-cleaving action of renin. A number of such substances are known including antirenin antibodies, pepstatin and naturally-occurring phospholipid compounds. European Patent Application No. 45,665 (published February 2, 1982) discloses a series of renin-inhibiting polypeptide derivatives of the formula

X-Y-Pro-Phe-His-A-B-Z-W

in which X may be hydrogen or an amino-protecting group, Y may be absent, B is a lipophilic amino acid residue, Z is an aromatic amino acid residue, W may be hydroxyl and A may be, inter alia,

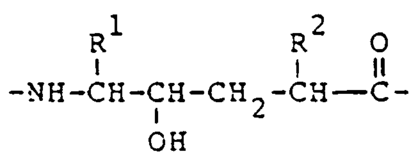

with each of $R^1$ and $R^2$ being a lipophilic or aromatic side chain. According to the definitions set forth in this published patent application, it is not contemplated that either A or Z could be statine or that B could be lysine.

European Patent Application No. 77,028 (published April 20, 1983) discloses a series of renin-inhibiting polypeptide compounds having a non-terminal statine or statine derivative residue. Included within this series are compounds having a phenylalanine-histidine-statine sequence.

European Patent Application 132,304 also discloses the use of statine containing polypeptides as renin-inhibiting antihypertensive agents, and European Patent Application 114,993 discloses polypeptides containing cyclostatine, useful as renin-inhibiting antihypertensive agents.

Further relevant prior art is disclosed in European Patent Applications 212903 and 220665 which also relate to renin-inhibiting antihypertensive polypeptides. Those of the former contain homocyclostatine residues which may or may not be located at the C-terminus, whilst those of the latter may contain a C-terminal cyclostatine or homocyclostatine residue although only polypeptides containing cyclostatine residues are exemplified.

The novel peptides of the present invention are of the formula

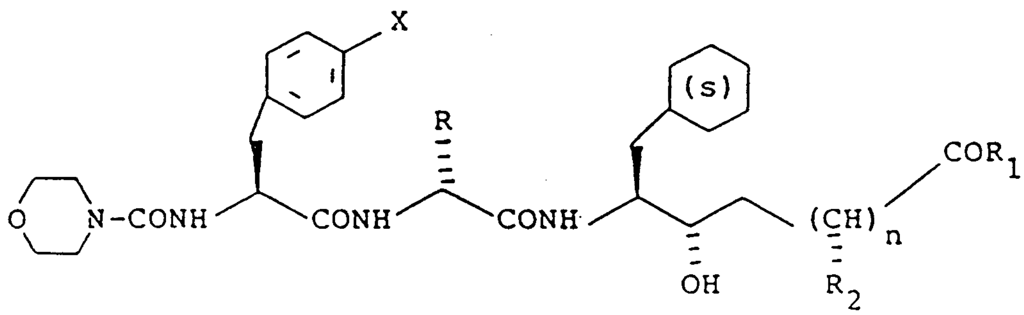

and a pharmaceutically acceptable salt thereof wherein X is hydrogen, methoxy or hydroxy; R is alkyl of one to six carbon atoms, imidazol-4-ylmethyl or methylthiomethyl; n is 1; $R_1$ is amino, alkylamino of one to five carbon atoms, alkoxy of one to three carbon atoms or 2-alkoxycarbonylpyrrolidin-1-yl, said alkoxy having from one to three carbon atoms; and $R_2$ is alkyl having from three to four carbon atoms, $-CH_2C(Cl)=CH_2$, $-CH_2CH=CHCH_2N_3$, $-(CH_2)_4NH_2$, $-CH_2C(Br)=CH_2$, $-CH_2CH=CHCl$, $-CH_2CH=C(CH_3)_2$, $-CH_2CH=CHCH_3$ or $-CH_2C{\equiv}CH$.

A preferred group of compounds are those where X is hydrogen and R is alkyl having three to four carbon atoms. Especially preferred in this group are the compounds where R is n-butyl, $R_1$ is methylamino and $R_2$ is i-butyl or $-CH_2C(Cl)=CH_2$.

A second preferred group of compounds are those where X is hydrogen and R is imidazol-4-ylmethyl. Especially preferred within this group are the compounds where $R_1$ is methylamino and $R_2$ is i-butyl.

The present invention also includes the use of a compound of the present invention for the manufacture of a medicament for treating hypertension in a mammal, and a pharmaceutical composition comprising an antihypertensive effective amount of a compound of the present invention and a pharmaceutically acceptable diluent or carrier.

As previously indicated, the present invention embraces pharmaceutically acceptable salts of the biologically active compounds. Such salts are those which are non-toxic at the dosages administered. Since compounds of the invention may contain basic groups, acid addition salts are possible. Pharmaceutically acceptable acid addition salts include e.g. the hydrochloride, hydrobromide, hydroiodide, sulfate, bisulfate, phosphate, acid phosphate, acetate, lactate, maleate, mesylate, fumarate, citrate, acid citrate, tartrate, bitartrate, succinate, gluconate and saccharate salts.

In the interest of brevity, the commonly accepted abbreviated name of the individual amino acids have been employed where possible. For example, the amino acid phenylalanine is abbreviated as Phe, histidine as His, lysine as Lys, and norleucine as Nle, etc. The amino protecting group t-butoxycarbonyl is abbreviated as Boc, benzyloxycarbonyl as CBZ and N-t-butoxycarbonyl on the imidazole of histidine as imBoc.

Homocyclostatine is of the formula

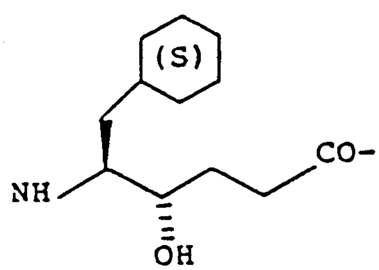

This structure is abbreviated as homo-C-Sta.

All the natural amino acid contained in the structures of the instantly claimed compounds are of the L configuration, the naturally occurring configuration, unless otherwise noted.

The compounds of this invention exhibit antihypertensive activity in vivo in mammals, including humans. At least a substantial portion of this actvity results from their ability to inhibit the cleavage of angiotensinogen by renin. Although we do not wish to be limited by the following theory of mechanism, it is likely

that the mechanism of the renin-inhibiting activity of the compounds of the invention is their selective binding (as compared to angiotensinogen) to renin. The compounds of the invention exhibit an enzyme-inhibiting activity that is selective for renin. Because of their low molecular weights they exhibit favorable solubilising characteristics in aqueous media, thus making oral administration feasible, and can be synthesized at a commercially realistic cost. The compounds of the present invention are also useful against congestive heart failure.

The compounds of the invention may be prepared by methods familiar to those skilled in the art. The basic sub-unit of the preferred chemical synthesis is the acylation of the unprotected alpha-amino group of an amino acid residue with an amino acid having an activated (for acylation purposes) carboxylic function and a suitable protecting group bonded to its own alpha-nitrogen to form a peptide bond between the two amino acid residues, followed by the removal of said protecting group. This synthesis sub-unit of coupling-deblocking is performed repeatedly to build up the polypeptide, starting from the C-terminal end as described herein. The amino acids utilized to synthesize the compounds of the present invention are commercially available (as free acids, salts or esters, etc.) in both alpha-amino protected and alpha-amino unprotected forms.

The activity of the compounds of the present invention as inhibitors of the angiotensinogen-cleaving activity of renin may be determined by studying their ability to inhibit the angiotensinogen-cleaving activity of renin in vitro.

The compounds of the present invention can be administered as antihypertensive agents by either the oral or parental routes of administration, with the former being preferred for reasons of patient convenience and comfort. In general, these antihypertensive compounds are normally administered orally in dosage ranging from about 0.5 mg to about 50 mg per kg of body weight per day and 0.1 mg to about 5 mg per kg of body weight per day when given parenterally; variations will necessarily occur depending upon the condition of the subject being treated and the particular compound being administered. Typically, treatment is commenced at a low daily dosage and increased by the physician only if necessary. It is to be noted that these compounds may be administered in combination with pharmaceutically acceptable carriers by either of the routes previously indicated, and that such administration can be carried out in both single and multiple dosages.

The novel compounds of the invention can be orally administered in a wide variety of different dosage forms, i.e., they may be formulated with various pharmaceutically acceptable inert carrier in the form or tablets, capsules, lozenges, troches, hard candies, powders, sprays, aqueous suspensions, elixirs, syrups and the like. Such carriers included solid diluents or fillers, sterile aqueous media and various non-toxic organic solvents, etc. Moreover, such oral pharmaceutical formulations cab be suitably sweetened and/or flavored by means of various agents of the type commonly employed for such purposes. In general, the compounds of this invention are present in such oral dosage forms at concentration levels ranging from about 0.5% to about 90% by weight of the total composition, in amounts which are sufficient to provide the desired unit dosages.

For purposes of oral administration, tablets containing various excipients such as sodium citrate, calcium carbonate and calcium phosphate may be employed along with various disintegrants such as starch and preferably potato or tapioca starch, alginic acid and certain complex silicate, together with binding agents such as polyvinylpyrrolidone, sucrose, gelatin and acacia. Additionally, lubricating agents such as magnesium stearate, sodium lauryl sulfate and talc are compositions of a similar type may also be employed as fillers in soft and hard-filled gelatin capsules; included lactose or milk sugar as well as high molecular weight polyethylene glycols. When aqueous suspensions and/or elixirs are desired of oral administration, the essential active ingredient therein may be combined with various sweetening or flavoring agents, coloring matter or dyes and, if so desired, emulsifying and/or as water, ethanol, propylene glycol, glycerin and various like combinations thereof.

The following examples illustrate the invention but are not to be construed as limiting the same.

High performance liquid chromatography (HPLC) was performed with the following conditions: 214 nm detection, 4.6 x 250 mm Dupont Zorbax C-8 column at 1.5 ml/min. TLC systems are abbreviated as follows: System A = ethyl acetate-hexane, respectively, in the ratio indicated, on silica; System B = ether-hexane, respectively, in the ratio indicated, on silica; System C = 18/2/1 $HCC1_3$-EtOH-HOAc on silica, System D = 9:2:1 chloroform-ethanol-acetic acid.

EXAMPLE 1

MorpholinocarbonylPheHis-2-isobutylhomo-C-Sta N-methylamide (X=H, n=1, R=imidazol-4-ylmethyl, $R_1$ = $NHCH_3$ and $R_2$ = $-CH_2CH(CH_3)_2$)

A. di-t-butoxycarbonyl histidine benzyl ester

$N_\alpha$-t-Boc-$N_\pi$-Boc-L-histidine (113.4 g) was dissolved in 800 ml dry dimethylformamide and the stirred solution treated at 0 °C. with 43.9 g anhydrous potassium carbonate and 37.8 ml benzyl bromide. The mixture was stirred in an ice bath which was allowed to reach 20 °C. overnight. The suspension was filtered through Celite which was washed with ether, the filtrates were concentrated and the residue dissolved in 700 ml ethyl acetate. This solution was washed with 2 x 200 ml of 1M aqueous lithium chloride, 100 ml of 4M aqueous lithium chloride, 2 x 100 ml 1N sodium hydroxide solution, water, brine, dried over magnesium sulfate and concentrated giving an oily solid which was stirred vigorously with 500 ml hexanes. The filtered solid was washed at 25 °C. with 2 x 100 ml hexane and dried giving 120.5 g (85.5%) of the title substance as a colorless solid, m.p. 97-99.5 °C., $[alpha]_D^{25}$ -6.4 ° (c=1.09, $CHCl_3$), TLC Rf 0.35 in 1:1 ethyl acetate-hexane. Material twice recrystallized from 1:4 ethyl acetate-hexane showed m.p. 99-106 °C. and $[alpha]_D^{23}$ -6.6 ° (c=1.25, $CHCl_3$).

B. histidine benzyl ester hydrochloride

The product of Example 1A was dissolved in a solution of 56 g of anhydrous hydrogen chloride in 400 ml p-dioxane. The suspension was stirred at 25 °C. for 24 hours. The mixture was concentrated, the residue washed with three portions of ether on the funnel and dried at 56 °C. in vacuo for 10 hours giving 69.5 g of a colorless solid, $[alpha]_D^{25}$ +6.4 ° (c=2.865, MeOH) (reported, Org. Prep. Proc. Int'l. 1970, 255, $[alpha]_D$ =6.54 °).

C. morpholinocarbonylPheHis benzyl ester

The product of Example 1B (1.37 g) was dissolved in 5 ml dichloromethane, cooled to 0 °C. and treated sequentially with 555 ul (1.0 equiv) triethylamine, 1.21 g (1.05 equiv) morpholinocarbonylPhe, 928 mg of hydroxybenzotriazole hydrate and 780 mg dicyclohexylcarbodiimide. This mixture was stirred in an ice bath which was allowed to achieve 20 °C. overnight. The mixture was filtered, the solids washed with dichloromethane and the filtrates were washed with 1N sodium hydroxide solution (2x), aqueous bicarbonate, brine, dried over magnesium sulfate and concentrated giving 1.8 g of a light yellow solid which was triturated with ether and dried to give 1.68 g (67%) of the title substance as a yellow solid.

D. morpholinocarbonylPheHis(imBoc) benzyl ester

The product of Example 1C (1.68 g) was dissolved in 30 ml p-dioxane and 15 ml water and the pH was raised to 11 by addition of 1N sodium hydroxide solution. Di-t-butyldicarbonate (850 ul) was added and the pH was maintained between 9 and 11 with added base. After 45 minutes another 450 ul of di-t-butyldicarbonate was added and the pH was maintained near 10.5. After 1.5 hours total, the pH was adjusted to 5 with 1N hydrochloric acid, the mixture was partially concentrated to remove the p-dioxane and the solution was extracted with ethyl acetate (4 x 100 ml). The combined organic layers were washed with 1N sodium hydroxide solution, aqueous bicarbonate, dried over sodium sulfate and concentrated giving 1.85 g of an oily foam which was chromatographed on 60 g silica eluting with 2%, 4%, 6% and 8% ethanol in dichloromethane, giving after concentration of the appropriate fractions 1.05 g of the title substance as a pale yellow foam, TLC Rf 0.13 in ethyl acetate.

E. morpholinocarbonylPheHis(imBoc)

A solution of 485 mg of the product of Example 1D in 25 ml 10:1 methanol-acetic acid was shaken with 200 mg 10% Pd/C for 30 minutes at 50 p.s.i. hydrogen pressure. The mixture was filtered through Celite which was washed with methanol-acetic acid, the filtrates concentrated, the residue coevaporated with ether and dried giving 361 mg (87%) of the title substance, Rf 0.25 in 18:2:1 chloroform-ethanol-acetic acid, as an off-white foam.

F. morpholinocarbonylPheHis(imBoc)-2-i-butylhomo-C-Sta lactone

A solution of 213 mg 2-isobutylhomo-C-Sta lactone hydrochloride (EPO Publication No. 0212903(A2) in 1.5 ml dichloromethane was treated sequentially at 0 ° C. with 126 ul triethylamine, 361 mg of the product of Example 1E, 161 mg 1-hydroxybenzotriazole and 144 mg dicyclohexylcarbodiimide. The mixture was stirred overnight during which time the temperature rose to 20 ° C. The mixture was filtered, the precipitate washed with dichloromethane, the filtrates concentrated, and the residue dissolved in ethyl acetate. After being stirred for 15 minutes, the resulting suspension was filtered, and the filtrate was washed with 1N sodium hydroxide solution (2x), brine, dried over magnesium sulfate and concentrated giving 754 mg of an off-white foam which was chromatographed on 35 g silica packed in 0.5% ethanol-dichloromethane. The column was eluted with 0.5%, 1%, 2% and 4% ethanol-dichloromethane and the appropriate fractions were concentrated giving 396 mg (74%) of the title substance as a colorless foam, TLC Rf 0.63 in 18/2/1 chloroform-ethanol-acetic acid.

G. morpholinocarbonylPheHis-2-i-butylhomo-C-Sta N-methylamide

The product of Example 1F (361 mg) was dissolved in methanol (3 ml) and the resulting solution was saturated at 0 ° C. with anhydrous methylamine. The flask was stoppered and the solution was allowed to stand at 25 ° C. for 70 minutes. The solvent and excess methylamine were removed at reduced pressure and the resulting powder was chromatographed on 20 g silica packed in 4% ethanol-dichloromethane. The column was eluted with 600 ml each of 4%, 6%, 12% and 15% ethanol-dichloromethane. Concentration of the appropriate fractions gave 296 mg (84%) of the title substance as a colorless powder, HPLC in 50/50 acetonitrile-buffer 3.23 minutes.

1H NMR (DMSO-d6, 300 mHz, δ, ppm, partial) 0.86 and 0.90 (d, 6H total), 0.97-1.80 (m, ca. 15H total), 2.40 (shoulder on DMSO peak), 2.51 (d, $NCH_3$), 2.66-3.0 (m, ca. 4H), 3.0-3.72 (m), 4.15 (m, 1H), 4.37 (m, 1H), 6.80 and 7.50 (s, 1H ea, imidazolyl CH), 7.60 and 8.47 (m, 1H ea). FAB-MS [thioglycerol, m/e (rel. intensity], 233(39), 250(14), 261(16), 283(17), 405(10), 642(16), 696(100, MH+), 697(47), 698(14).

EXAMPLE 2

MorpholinocarbonylPheNle-2-i-butylhomo-C-Sta N-methylamide (X=H, n=1, R=n-$C_4H_9$, $R_1$=$NHCH_3$ and $R_2$ = $-CH_2CH(CH_3)_2$)

A. morpholinocarbonylPheNle-2-i-butylhomo-C-Sta lactone

According to the general procedure for preparation and purification of the product of Example 1F (replacing His with Nle), 52 mg of 2-isobutyl-homo-C-Sta lactone hydrochloride was dissolved in 0.5 ml dichloromethane and treated with 34 ul triethylamine, 67 mg of morpholino-carbonylPheNle, 39 mg HBT and 35 mg DCC. After analogous reaction and isolation (chromatography was not necessary in this case), 103 mg of the title substance was isolated as a colorless foam, TLC Rf 0.69 in 18/2/1 $HCCl_3$-ethanol-HOAc.

B. morpholinocarbonylPheNle-2-i-butylhomo-C-Sta N-methylamide

According to the procedure for preparation and purification of the product of Example 1G, 102 mg of the product of Example 2A gave 77 mg (72%) of the title substance as a colorless powder, TLC Rf 0.5 in 18/2/1 $HCCl_3$-ethanol-HOAc, HPLC 2.85 minutes in 70/30 acetonitrile-buffer.

1H NMR (DMSO-d6, 300 mHz, δ, ppm, partial): 0.82-1.0 (m, 9-10H), 1.0-1.9 (m, ca. 24H), 2.46 (m, 1H), 2.57 (d, 3H, $NCH_3$), 2.86 (dd, 1H), 3.02 (dd, 1H), 3.26 (m, 4H), 3.51 (m, 4H), 3.78 (m, 1H), 4.22 (m, 2H), 4.59 (d, 1H), 6.70 (d, 1H), 7.14-7.44 (m, ca. 6H), 7.63 (m, 1H), 8.00 (d, 1H); FAB-MS [thioglycerol, m/e (rel. intensity)], 217(18), 233(61), 234(11), 250(13), 264(10), 268(17), 281(12), 299(21), 381(12), 394(14), 654(39),

655(25), 656(17), 670(12), 672(100, MH+), 673(40), 674(11).

EXAMPLE 3

MorpholinocarbonylPheNle-2-(2'-chloro-2'-propenyl)homo-C-Sta N-methylamide (X=H, R=$CH_3(CH_2)_3$-, $R_1$ =$NHCH_3$ and $R_2$ = -$CH_2C(Cl)=CH_2$)

A. morpholinocarbonylPheNle-2-(2'-chloro-2'-propenyl)homo-C-Sta lactone

The procedure of Example 1F was repeated using 57 mg of 2-(2'-chloro-2'-propenyl)homo-C-Sta lactone in 15 ml of dichloromethane, 19.5 $\mu$l of N-methylmorpholine, 69.8 mg of morpholinocarbonylPheNle, 23.9 mg of 1-hydroxybenzotriazole and 36.5 mg of cyclohexylcarbodiimde to give 88 mg of a foam which was chromatographed on 10 g of silica packed in chloroform. The column was eluted with chloroform and 99% chloroform-methanol (v:v), and the appropriate fractions combined and concentrated to give 43 mg of product.

B. morpholinocarbonylPheNle-2-(2'-chloro-2'-propenyl)homo-C-Sta N-methylamide

The product of Example 3A (43 mg) was dissolved in methanol (5 ml) and the resulting solution was saturated at 0°C with anhydrous methylamine. The flask was stoppered and the solution was allowed to stand at 25°C for 70 minutes. The solvent and excess methylamine were removed at reduced pressure and the resulting powder was triturated with ether to give 33 mg of the title substance as a colorless powder, $^1$H-NMR (DMSO-$d_6$, 300 mHz, $\delta$, ppm, partial): 2.5 (d, $NCH_3$), 3.4 (m, 4H), 3.6 (m, 4H); 5.15 (m, 2H).

EXAMPLE 4

MorpholinocarbonylPheNle-2-(4'-azido-2'-butenyl)homo-C-Sta N-methylamide (X=H, R=$CH_3(CH_2)_3$-,$R_1$ =$NHCH_3$ and $R_2$ =-$CH_2CH=CHCH_2N_3$)

A. morpholinocarbonylPheNle-2-(4'-azido-2'-butenyl)homo-C-Sta lactone

A solution of 97 mg 2-(4'-azido-2'-butenyl)homo-C-Sta lactone hydrochloride in 20 ml dichloromethane was treated sequentially at 0°C with 28 $\mu$l N-methylmorpholine, 110.6 mg of morpholinocarbonylPheNle, 38.2 mg 1-hydroxybenzotriazole and 58.3 mg dicyclohexylcarbodiimide. The mixture was stirred overnight during which time the temperature rose to 20°C. The mixture was filtered, the precipitate washed with dichloromethane, the filtrates concentrated and the residue dissolved in ethyl acetate. After being stirred for 15 minutes, the resulting suspension was filtered, and the filtrate was washed with 1N sodium hydroxide solution (2x), brine, dried over magnesium sulfate and concentrated giving 221 mg of an off-white foam which was chromatographed on 22 g silica packed in chloroform. The column was eluted with 99% chloroform-methanol and the appropriate fractions were concentrated giving 121 mg of the title substance as a colorless foam.

B. morpholinocarbonylPheNle-2-(4'-azido-2'-butenyl)homo-C-Sta N-methylamide

The product of Example 4A (121 mg) was dissolved in methanol (10 ml) and the resulting solution was saturated at 0°C with anhydrous methylamine. The flask was stoppered and the solution was allowed to stand at 25°C for 70 minutes. The solvent and excess methylamine were removed at reduced pressure and the resulting powder was triturated with ether to give 88 mg of the title substance as a colorless powder, $^1$H-NMR (DMSO-$d_6$, 300 mHz, $\delta$, ppm, partial): 2.5 (d, $NCH_3$), 3.4 (m, 4H), 3.6 (m, 4H).

EXAMPLE 5

MorpholinocarbonylPheNle-2-(4'-amino-1'-butyl)homo-C-Sta N-methylamide (X=H, R=$CH_3(CH_2)_3$, $R_1$ =$NHCH_3$ and $R_2$ = -$(CH_2)_4NH_2$)

The product of Example 4B (58 mg) was hydrogenated at 50 psi in methanol in the presence of 20 mg of 5% palladium hydroxide on carbon for 19 hours at 25°C. The reaction after filtration was evaporated to dryness to afford (after ether trituration) 44 mg of the title compound. $^1$H-NMR (DMSO-$d_6$, 300 mHz, $\delta$, ppm,

partial): 2.5 (d, $NCH_3$), 3.4 (m, 4H), 3.6 (m, 4H).

EXAMPLE 6

MorpholinocarbonylPheNle-2-(2'-bromo-2'-propenyl)homo-C-Sta N-methylamide (X = H, R = $CH_3)CH_2)_3$-, $R_1$ = $NHCH_3$ and $R_2$ = $CH_2C(Br) = CH_2$)

A. morpholinocarbonylPheNle-2-(2'-bromo-2'-propenyl)homo-C-Sta lactone

A solution of 78 mg 2-(2'-bromo-2'-propenyl)homo-C-Sta lactone hydrochloride in 20 ml dichloromethane was treated sequentially at 0 ° C with 22 µl N-methylmorpholine, 78.2 mg of morpholinocarbonylPheNle, 27 mg 1-hydroxybenzotriazole and 41.2 mg dicyclohexylcarbodiimide. The mixture was stirred overnight during which time the temperature rose to 20 ° C. The mixture was filtered, the precipitate washed with dichloromethane, the filtrate concentrated, and the residue dissolved in ethyl acetate. After being stirred for 15 minutes, the resulting suspension was filtered, and the filtrate was washed with 1N sodium hydroxide solution (2x), brine, dried over magnesium sulfate and concentrated giving 190 mg of an off-white foam which was chromatographed on 10 g silica packed in chloroform. The column was eluted and the appropriate fractions were concentrated giving 78 mg of the title substance as a colorless foam, TLC Rf 0.85 in 9/1 chloroform-methanol.

B. morpholinocarbonylPheNle-2-(2'-bromo-2'-propenyl)homo-C-Sta N-methylamide

The product of Example 6A (78 ml) was dissolved in methanol (10 ml) and the resulting solution was saturated at 0 ° C with anhydrous methylamine. The flask was stoppered and the solution was allowed to stand at 25 ° C For 70 minutes. The solvent and excess methylamine were removed at reduced pressure and the resulting powder was triturated with either to give 55 mg of the title substance as a colorless powder, $^1$H-NMR (DMSO-$d_6$, 300 mHz, δ, ppm, partial): 2.5 (d, $NCH_3$), 3.4 (m, 4H), 3.6 (m, 4H), 5.5 (m, 2H).

EXAMPLE 7

MorpholinocarbonylPheNle-2-(3'-chloro-2'-propenyl)homo-C-Sta N-methylamide (X = H, R = $CH_3(CH_2)_3$, $R_1$ = $NHCH_3$ and $R_2$ = -$CH_2CH = CHCl$)

A. morpholinocarbonylPheNle-2-(3'-chloro-2'-propenyl)homo-C-Sta lactone

A solution of 136 mg 2-(3'-chloro-2'-propenyl)homo-C-Sta lactone hydrochloride in 15 ml dichloromethane was treated sequentially at 0 ° C with 21.4 µl N-methylmorpholine, 76 mg of morpholinocarbonylPheNle, 26.2 mg 1-hydroxybenzotriazole and 40 mg dicyclohexylcarbodiimide. The mixture was stirred overnight during which time the temperature rose to 20 ° C. The mixture was filtered, the precipitate washed with dichloromethane, the filtrates concentrated and the residue dissolved in ethyl acetate. After being stirred for 15 minutes, the resulting suspension was filtered, and the filtrate was washed with 1N sodium hydroxide solution (2x), brine, dried over magnesium sulfate and concentrated giving 167 mg of an off-white foam which was chromatographed on 17 g silica packed in chloroform. The column was eluted with chloroform and 99% chloroform-methanol and the appropriate fractions were concentrated giving 103 mg of the title substance as a colorless foam, TLC Rf 0.9 in 9/1 chloroform-methanol.

B. morpholinocarbonylPheNle-2-(3'-chloro-2'-propenyl)homo-C-Sta N-methylamide

The product of Example 7A (103 mg) was dissolved in methanol (10 ml) and the resulting solution was saturated at 0 ° C with anhydrous methylamine. The flask was stoppered and the solution was allowed to stand at 25 ° C for 70 minutes. The solvent and excess methylamine were removed at reduced pressure and the resulting powder was triturated with ether to give 74 mg of the title substance as a colorless powder, $^1$H-NMR (DMSO-$d_6$, 300 mHz, δ, ppm, partial): 2.5 (d, $NCH_3$), 3.4 (m, 4H), 3.6 (m, 4H), 5.6-6.4 (m, 2H).

EXAMPLE 8

MorpholinocarbonylPheNle-2-(3',3'-dimethyl-2'-propenyl)homo-C-Sta N-methylamide (X = H, R = $CH_3(CH_2)_3$-, $R_1$ = $NHCH_3$ and $R_2$ = $-CH_2CH=C(CH_3)_2$)

A. morpholinocarbonylPheNle-2-(3',3'-dimethyl-2'-propenyl)homo-C-Sta lactone

A solution of 132 mg 2-(3',3'-dimethyl-2'-propenyl)homo-C-Sta lactone hydrochloride in 15 ml dichloromethane was treated sequentially at 0 °C with 21.7 µl N-methylmorpholine, 77.3 mg of morpholinocarbonylPheNle, 26.7 mg 1-hydroxybenzotriazole and 40.7 mg dicyclohexylcarbodiimide. The mixture was stirred overnight during which time the temperature rose to 20 °C. The mixture was filtered, the precipitate washed with dichloromethane, the filtrates concentrated, and the residue dissolved in ethyl acetate. After being stirred for 15 minutes, the resulting suspension was filtered, and the filtrate was washed with 1N sodium hydroxide solution (2x), brine, dried over magnesium sulfate and concentrated giving 150 mg of an off-white foam which was chromatographed on 15 g silica packed in chloroform. The column was eluted with chloroform and 99% chloroform-methanol and the appropriate fractions were concentrated giving 89 mg of the title substance as a colorless foam, TLC Rf 0.6 in 9/1 chloroform-methanol.

B. morpholinocarbonylPheNle-2-(3',3'-dimethyl-2'-propenyl)homo-C-Sta N-methylamide

The product of Example 8A (89 mg) was dissolved in methanol (10 ml) and the resulting solution was saturated at 0 °C with anhydrous methylamine. The flask was stoppered and the solution was allowed to stand at 25 °C for 70 minutes. The solvent and excess methylamine were removed at reduced pressure and the resulting powder was triturated with ether to give 65 mg of the title substance as a colorless powder, $^1$H-NMR (DMSO-$d_6$, 300 mHz, δ, ppm, partial): 1.8 (br. s, 6H), 2.5 (d, $NCH_3$), 3.4 (m, 4H), 3.6 (m, 4H).

EXAMPLE 9

MorpholinocarbonylPheNle-2-(2'-butenyl)homo-C-Sta N-methylamide (X = H, R = $CH_3(CH_2)_3$, $R_1$ = $NHCH_3$ and $R_2$ = $CH_2CH=CHCH_3$)

A. morpholinocarbonylPheNle-2-(2'-butenyl)homo-C-Sta lactone

A solution of 247 mg 2-(2'-butenyl)homo-C-Sta lactone hydrochloride in 15 ml dichloromethane was treated sequentially at 0 °C with 26.3 µl N-methylmorpholine, 93.4 mg of morpholinocarbonylPheNle, 32.3 mg 1-hydroxybenzotriazole and 49.2 mg dicyclohexylcarbodiimide. The mixture was stirred overnight during which time the temperature rose to 20 °C. The mixture was filtered, the precipitate washed with dichloromethane, the filtrates concentrated, and the residue dissolved in ethyl acetate. After being stirred for 15 minutes, the resulting suspension was filtered, and the filtrate was washed with 1N sodium hydroxide solution (2x), brine, dried overmagnesium sulfate and concentrated giving 168 mg of an off-white foam which was chromatographed on 10 g silica packed in chloroform. The column was eluted with 99% chloroform-methanol and the appropriate fractions were concentrated giving 96 mg of the title substance as a colorless foam, TLC Rf 0.7 in 9/1 chloroform-methanol.

B. morpholinocarbonylPheNle-2-(2'-butenyl)homo-C-Sta N-methylamide

The product of Example 9 A (96 mg) was dissolved in methanol (10 ml) and the resulting solution was saturated at 0 °C with anhydrous methylamine. The flask was stoppered and the solution was allowed to stand at 25 °C for 70 minutes. The solvent and excess methylamine were removed at reduced pressure and the resulting powder was triturated with ether to give 66 mg of the title substance as a colorless powder, $^1$H-NMR (DMSO-$d_6$, 300 mHz, δ, ppm, partial): 1.8 (br.s, 3H); 2.5 (d, $NCH_3$), 3.4 (m, 4H), 3.6 (m, 4H).

EXAMPLE 10

MorpholinocarbonylPheNle-2-(propargyl)homo-C-Sta N-methylamide (x = H, R = $CH_3(CH_2)_3$-, $R_1$ = $NHCH_3$ and $R_2$ = $CH_2C{\equiv}CH$)

A. morpholinocarbonylPheNle-2-(3'-propargyl) homo-C-Sta lactone

A solution of 193 mg 2-(3'-propargyl)homo-C-Sta lactone hydrochloride in 15 ml dichloromethane was treated sequentially at 0 °C with 22.4 µl N-methylmorpholine, 83.2 mg of morpholinocarbonylPheNle, 28.7 mg 1-hydroxybenzotriazole and 43.8 mg dicyclohexylcarbodiimide. The mixture was stirred overnight during which time the temperature rose to 20 °C. The mixture was filtered, the precipitate washed with dichloromethane, the filtrates concentrated, and the residue dissolved in ethyl acetate. After being stirred for 15 minutes, the resulting suspension was filtered, and the filtrate was washed with 1N sodium hydrodie solution (2x), brine, dried over magneisum foam which was chormatographed on 10 g silica packed in chloroform. The column was eluted with 99% chloroform-methanol and the appropriate fractions were concentrated giving 98 mg of the title substance as a colorless foam, TLC Rf 0.6 in9/1 chloroform-methanol.

B. morpholinocarbonylPheNle-2-(3'-propargyl)homo-C-Sta N-methylamide

The product of Example 10A (98 mg) was dissolved in methanol (10 ml) and the resulting solution was saturated at 0 °C with anhydrous methylamine. The flask was stoppered and the solution was allowed to stand at 25 °C for 70 minutes. The solvent and excess methylamine were removed at reduced pressure and the resulting powder was triturated with ether to give 90 mg of the title substance as a colorless powder, $^1H$-NMR (DMSO-$d_6$, 300 mHz, δ, ppm, partial): 2.3 (m, 2H), 2.5 (d, $NCH_3$), 3.4 (m, 4H), 3.6 (m, 4H).

PREPARATION 1

MorpholinocarbonylPheNle

A. Norleucine benzyl ester

According to the general procedure outlined in J. Med. Chem. 1986, Vol. 30, p. 3575, 15.0 g norleucine (Nle) was mixed with 200 ml benzyl alcohol and cooled to 0 °C. Thionyl chloride (25 ml) was added dropwise over 15 minutes and the mixture was slowly heated to 90 °C. with a fierce evolution of $SO_2$ occurring at about 50 °C. After 2 hours at 90 °C. the mixture was cooled to 0 °C. and 25 ml more thionyl chloride was added. The mixture was then heated again at 90 °C. for 2 hours, cooled, diluted with 1.6 liters ether and stored overnight at 0 °C. The crystals which formed were filtered, washed with ether and dried to give 23.1 g of a damp solid which was recrystallized from 1:10 ethanol-ether, using 23 ml ethanol. The filtered and dried solid weighed 17.1 g, TLC Rf 0.25 in System C (the spotted plate was exposed to ammonia vapor and dried prior to elution).

B. (S)-2-Isocyanato-3-phenylpropionic acid benzyl ester

According to the procedure of Lombardino et al. (J. Med. Chem. 1964, 7, 97) 18.0 g L-phenylalanine benzyl ester hydrochloride in 150 ml toluene was stirred at reflux under an atmosphere of phosgene for 1.5H, cooled and concentrated to give a solid which was recrystallized from 120 ml hexane to give 16.1 g of colorless needles.
Anal. Calcd for $C_{17}H_{15}NO_3$: C, 72.59; H, 5.37; N, 4.98. Found: C, 72.32; H, 5.35; N, 4.92. MP 68-72 °C. $[alpha]_D^{23}$ -80.4 ° (c = 1.02, $CHCl_3$). IR ($CHCl_3$) 2250, 1750 $cm^{-1}$.

C. MorpholinocarbonylPhe benzyl ester

The product of Preparation 1B was dissolved in 5 ml dichloromethane, treated at 25 °C. with 930 ul morpholine and after 30 minutes the mixture was concentrated to a waxy solid which was recrystallized from hot 4:1 hexane-ethyl acetate, giving 1.92 g of the title substance, mp 87-89 °C. MS (chemical ionization, isobutane) 369 (MH + , base peak).

D. Morpholinocarbonyl Phe

The product of Preparation 1C (1.85 g) was dissolved in 30 ml absolute methanol and 5 ml acetic acid and shaken with 0.5 g 10% Pd/C for 1 hour under a 53 psi hydrogen atmosphere. The suspension was filtered, concentrated, co-evaporated three times with added toluene and dried to give 1.43 g of a colorless foam.

E. MorpholinocarbonylPheNle benzyl ester

Following the procedure for preparation and purification of the product of Example 1, 2.12 g of the product of Preparation 1A and 2.63 g of the product of Preparation 1C gave 3.30 g of the title substance as a colorless foam, TLC Rf 0.5 in ethyl acetate on silica, HPLC ret. time 3.27 minutes 97% of total absorption to 25 minutes in 70/30 MeCN-pH 2.1 0.1M phosphate.

F. MorpholinocarbonylPheNle

The product of Preparation 1D (3.3 g) was shaken in 35 ml methanol and 7 ml acetic acid with 1.0 g 10% Pd/C for 45 minutes, filtered through Celite, concentrated, co-evaporated several times with toluene and ether and dried to give 2.9 g of a colorless solid, TLC Rf 0.2 in System C.

PREPARATION 2

2R,4S,5S-6-Cyclohexyl-5-(t-butoxycarbonylamino)-2-(2'-chloro-2'-propenyl)-gamma-hexanolactone

To a tetrahydrofuran solution containing 37.5 mmol of lithium diethylamide at -70°C (prepared from 23.4 ml of 1.6M butyl lithium hexane and 4.26 g of diethyl amine in 50 ml of dry tetrahydrofuran) was added dropwise a solution of 4.67 g (15 mmol) of 4S,5S-6-cyclohexyl-5-(t-butoxycarbonylamino)-gamma hexanolactone in 25 ml of tetrahydrofuran. After 30 minutes at -78°C a solution of 3.64 g (16 mmol) of 2-chloro-3-iodopropene in 25 ml of tetrahydrofuran was added dropwise at -70°C. After 2 hours the reaction mixture was quenched with 10 ml of a saturated ammonium chloride solution added dropwise at -78°C, and the resulting mixture allowed to warm to room temperature. The solvent was removed in vacuo and the residue extracted with with diethyl ether. The ether solution was washed with a 10% citric acid solution, a saturated sodium bicarbonate solution and a brine solution. The ether solution was then dried over magnesium sulfate and concentrated to give 6.83 g of an oil, which was chromatographed on silica gel using ethyl acetate-hexane as the eluent. The fractions containing the product were combined and concentrated to give 2.38 g of the desired product.

The NMR spectrum ($CDCl_3$) showed absorption at 1.4 (9H, s) ppm.

PREPARATION 3

Employing the procedure of Preparation A, and using the appropriate starting reagents, the following intermediates were synthesized:

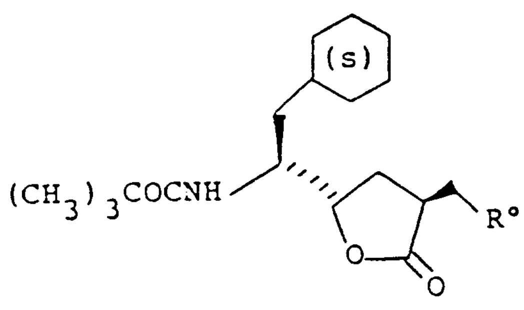

R°

$-CH=CH-CH_2Br$

$-C(Br)=CH_2$

$-CH=CHCl$

$-CH=C(CH_3)_2$

$CH=CHCH_3$

$C\equiv CH$

PREPARATION 4

2R,4S,5S-6-Cyclohexyl-5-(t-butoxycarbonylamino)-2-(4-azido-2-butenyl)-gamma-hexanolactone

A solution of 710 mg (1.6 mmol) of 2R,4S,5S-6-cyclohexyl-5-(t-butoxycarbonylamino)-2-(4'-bromo-2'-butenyl)gamma-hexanolactone and 986 mg (15.2 mmol) of sodium azide in 75 ml of dimethylsulfoxide-water (2:1; v:v) was allowed to stir overnight at room temperature. The reaction was poured into 500 ml of water and the product extracted with ethyl acetate. The extracts were combined and washed successively with water and a brine solution, and were dried over magnesium sulfate. The solvent was removed *in vacuo* to give 73 mg of the desired product.

PREPARATION 5

2R,4S,5S-6-Cyclohexyl-5-amino-2-(2'-chloro-2'-propenyl)-gamma-hexanolactone hydrochloride

A solution of 385 mg (1 mmol) of 2R,4S,5S-6-cyclohexyl-5-(t-butoxycarbonylamino)-2-(2'-chloro-2'-propenyl)-gamma-hexanolactone in 10 ml of 4.7N hydrogen chloride in dioxane was allowed to stir for 2 hours at room temperature. The solvent was removed *in vacuo* to give 331 mg of the desired amine hydrochloride.

PREPARATION 6

Using the procedure of Preparation 5, and employing the intermediates of Preparations 2, 3 and 4, the following intermediates were prepared:

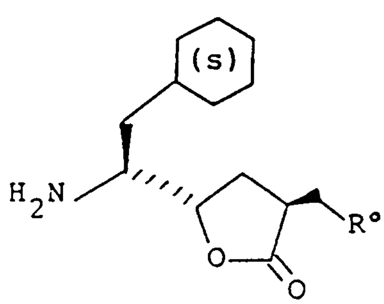

R°

$-C=CH_2$
Cl

$CH=CHCH_2N_3$

$-C=CH_2$
Br

$-CH=CHCl$

$-CH=C(CH_3)_2$

$CH=CHCH_3$

$C{\equiv}CH$

## Claims

**Claims for the following Contracting States : AT, BE, CH, DE, FR, GB IT, LI, LU, NL, SE**

**1.** A compound of the formula

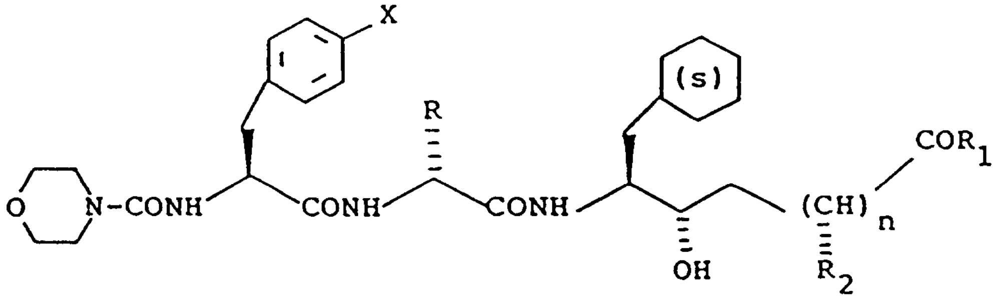

and a pharmaceutically acceptable salt thereof wherein X is hydrogen, methoxy or hydroxy; R is alkyl having one to six carbon atoms, imidazol-4-ylmethyl or methylthiomethyl; n is 1; $R_1$ is amino, alkylamino having from one to five carbon atoms, alkoxy having from one to three carbon atoms or 2-alkoxycarbonylpyrrolidin-1-yl said alkoxy having from one to three carbon atoms; and $R_2$ is alkyl having three to four carbon atoms, $-CH_2C(Cl)=CH_2$, $-CH_2CH=CH\text{-}CH_2N_3$, $-(CH_2)_4NH_2$, $-CH_2C(Br)=CH_2$, $-CH_2CH=CHCl$, $-CH_2CH=C(CH_3)_2$, $-CH_2CH=CHCH_3$ or $-CH_2C{\equiv}CH$.

**2.** A compound of claim 1 wherein X is hydrogen and R is alkyl having three to four carbon atoms.

**3.** The compound of claim 2 wherein R is n-butyl, $R_1$ is methylamino and $R_2$ is i-butyl.

**4.** A compound of claim 2 wherein R is n-butyl, $R_1$ is methylamino and $R_2$ is $-CH_2C(Cl)=CH_2$.

**5.** A compound of claim 1 wherein X is hydrogen and R is imidazol-4-ylmethyl.

**6.** A compound of claim 5 wherein $R_1$ is methylamino and $R_2$ is i-butyl.

**7.** A pharmaceutical composition comprising an antihypertensive effective amount of a compound according to claim 1 and a pharmaceutically acceptable diluent or carrier.

**Claims for the following Contracting States : ES, GR**

1. A process for preparing a compound of the formula

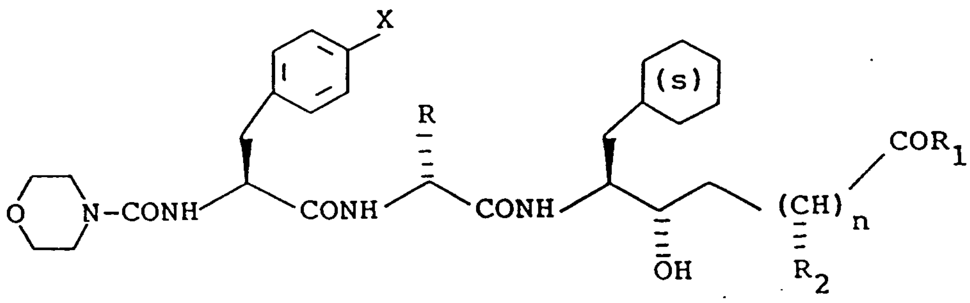

wherein X is hydrogen, methoxy or hydroxy; R is alkyl having one to six carbon atoms, imidazol-4-ylmethyl or methylthiomethyl; n is 1; $R_1$ is amino, alkylamino having from one to five carbon atoms, alkoxy having from one to three carbon atoms or 2-alkoxycarbonylpyrrolidin-1-yl said alkoxy having from one to three carbon atoms; and $R_2$ is alkyl having three to four carbon atoms, $-CH_2C(Cl)=CH_2$, $-CH_2CH=CH-CH_2N_3$, $-(CH_2)_4NH_2$, $-CH_2C(Br)=CH_2$, $-CH_2CH=CHCl$, $-CH_2CH=C(CH_3)_2$, $-CH_2CH=CHCH_3$ or $-CH_2C\equiv CH$, characterized by the formation of an amide bond, C(O)N, by dehydrative-coupling wherein the functional groups not involved in the reaction may be blocked followed by the optional steps of 1) treating a compound where $R_1$ is alkoxy having one to three carbon atoms with ammonia or an alkylamine having one to five carbon atoms to give the product where $R_1$ is amino or alkylamino having one to five carbon atoms, 2) reducing a compound where $R_2$ is $-CH_2-CH=CH-CH_2N_3$ to give the product where $R_2$ is $-(CH_2)_4NH_2$, 3) selectively removing the blocking groups and, if desired, making a pharmaceutically acceptable salt of the product.

2. The process of claim 1, wherein the amide bond is formed between the fragments

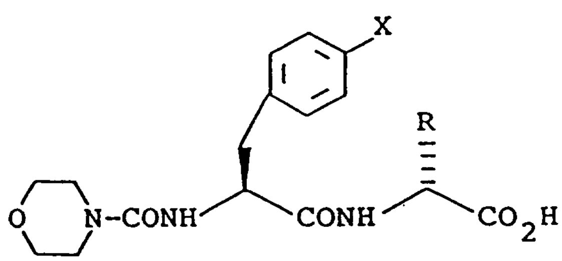

and

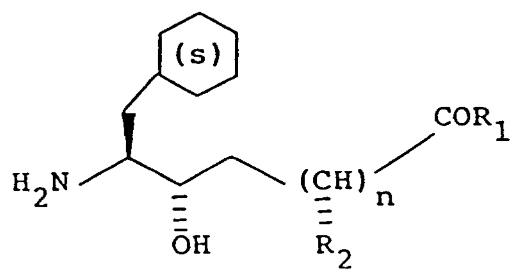

.

**3.** The process of claim 1, wherein the amide bond is formed between the fragments

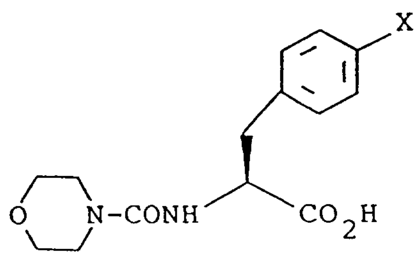

and

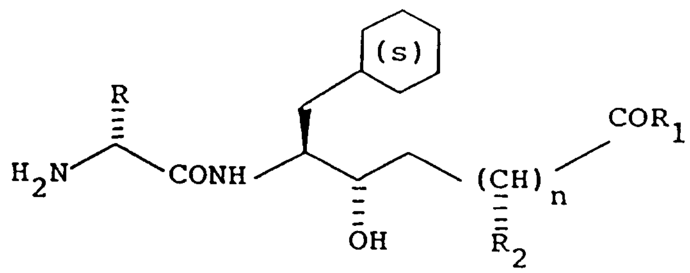

**4.** The process of claim 1, wherein the coupling reagent is 1-hydroxybenzotriazole and dicyclohexylcarbodiimide.

**5.** A process for preparing a compound of the formula

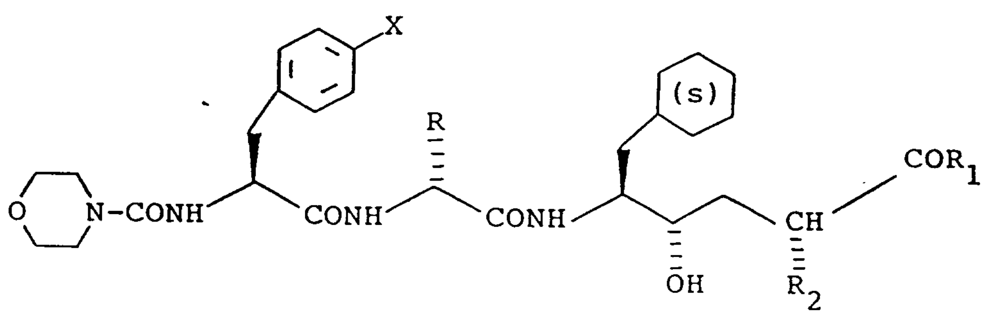

wherein X is hydrogen, methoxy or hydroxy; R is alkyl having one to six carbon atoms, imidazol-4-ylmethyl or methylthiomethyl; $R_1$ is amino, alkylamino having from one to five carbon atoms, alkoxy having from one to three carbon atoms or 2-alkoxycarbonylpyrrolidin-1-yl said alkoxy having from one to three carbon atoms; and $R_2$ is alkyl having three to four carbon atoms, $-CH_2C(Cl)=CH_2$, $-CH_2CH=CH-CH_2N_3$, $-(CH_2)_4NH_2$, $-CH_2C(Br)=CH_2$, $-CH_2CH=CHCl$, $-CH_2CH=C(CH_3)_2$, $-CH_2CH=CHCH_3$ or $-CH_2C\equiv CH$, characterized by reacting a compound of the formula

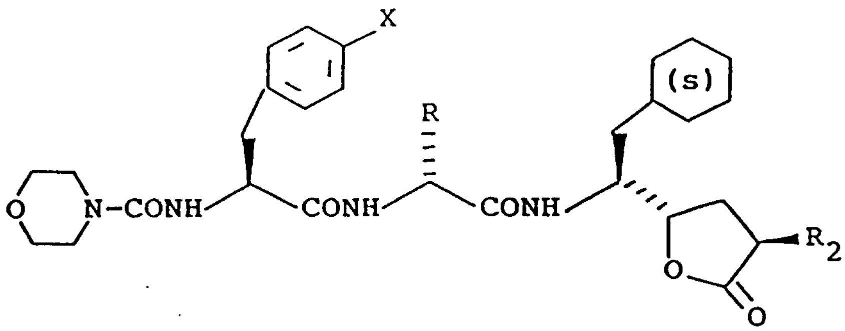

with ammonia, an alkanol having one to three carbon atoms, an alkylamine having one to five carbon atoms or 2-alkoxycarbonylpyrrolidine, said alkoxy having one to three carbon atoms wherein the functional groups not involved in the reaction may be blocked, followed by the optional steps of 1) reducing a compound where $R_2$ is $-CH_2-CH=CH-CH_2N_3$ to give the product where $R_2$ is $-(CH_2)_4NH_2$, 2) selectively removing the blocking groups and, if desired, making a pharmaceutically acceptable salt of the product.

## Patentansprüche
## Patentansprüche für folgende Vertragsstaaten : AT, BE, CH, DE, FR, GB, IT, LI, LU, NL, SE

**1.** Verbindung mit der folgenden Formel:

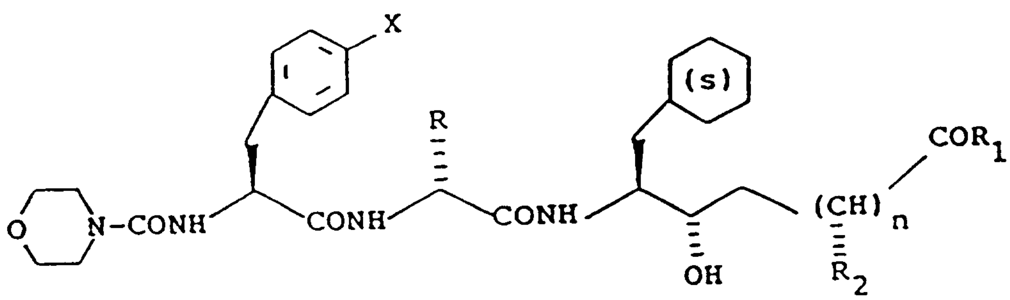

und ein pharmazeutisch geeignetes Salz davon, wobei X für Wasserstoff, Methoxy oder Hydroxy steht, R für Alkyl mit einem bis sechs Kohlenstoffatomen, Imidazol-4-ylmethyl oder Methylthiomethyl steht, n für 1 steht, $R_1$ für Amino, Alkylamino mit einem bis fünf Kohlenstoffatomen, Alkoxy mit einen bis drei Kohlenstoffatomen oder 2-Alkoxycarbonylpyrrolidin-1-yl steht, wobei das Alkoxy ein bis drei Kohlenstoffatome hat, und $R_2$ für Alkyl mit drei bis vier Kohlenstoffatomen, $-CH_2C(Cl)=CH_2$, $-CH_2CH=CH-CH_2N_3$, $-(CH_2)_4NH_2$, $-CH_2C(Br)=CH_2$, $-CH_2CH=CHCl$, $-CH_2CH=C(CH_3)_2$, $-CH_2CH=CHCH_3$ oder $-CH_2C{\equiv}CH$ steht.

**2.** Verbindung nach Anspruch 1, wobei X für Wasserstoff steht und R für Alkyl mit drei bis vier Kohlenstoffatomen steht.

**3.** Verbindung nach Anspruch 2, wobei R für n-Butyl, $R_1$ für Methylamino und $R_2$ für i-Butyl steht.

**4.** Verbindung nach Anspruch 2, wobei R für n-Butyl, $R_1$ für Methylamino und $R_2$ für $-CH_2C(Cl)=CH_2$ steht.

**5.** Verbindung nach Anspruch 1, wobei X für Wasserstoff und R für Imidazol-4-ylmethyl steht.

**6.** Verbindung nach Anspruch 5, wobei $R_1$ für Methylamino und $R_2$ für i-Butyl steht.

**7.** Pharmazeutische Zusammensetzung, die eine antihypertensiv wirksame Menge einer Verbindung nach Anspruch 1 und ein pharmazeutisch geeignetes Verdünnungsmittel oder Trägermittel enthält.

**Patentansprüche für folgende Vertragsstaaten : ES, GR**

1. Verfahren zur Herstellung einer Verbindung mit der folgenden Formel:

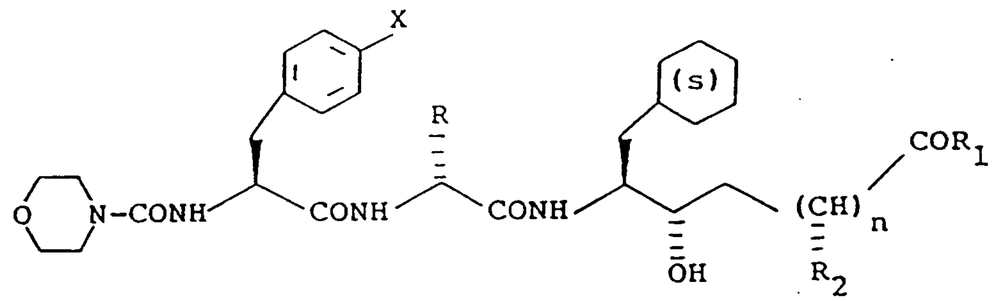

in der X für Wasserstoff, Methoxy oder Hydroxy steht, R für Alkyl mit einem bis sechs Kohlenstoffatomen, Imidazol-4-ylmethyl oder Methylthiomethyl steht, n für 1 steht, $R_1$ für Amino, Alkylamino mit einem bis fünf Kohlenstoffatomen, Alkoxy mit einem bis drei Kohlenstoffatomen oder 2-Alkoxycarbonylpyrrolidin-1-yl steht, wobei das Alkoxy ein bis drei Kohlenstoffatome hat, und $R_2$ für Alkyl mit drei bis vier Kohlenstoffatomen, $-CH_2C(Cl)=CH_2$, $-CH_2CH=CH-CH_2N_3$, $-(CH_2)_4NH_2$, $-CH_2C(Br)=CH_2$, $-CH_2CH=CHCl$, $-CH_2CH=C(CH_3)_2$, $-CH_2CH=CHCH_3$ oder $-CH_2C\equiv CH$ steht, gekennzeichnet durch die Bildung einer Amid-Bindung, C(O)N , durch dehydratisierende Kupplung, wobei die nicht an der Umsetzung beteiligten funktionellen Gruppen geschützt sein können, gefolgt von den folgenden wahlfreien Schritten, nämlich 1) Behandlung einer Verbindung, bei der $R_1$ für Alkoxy mit einem bis drei Kohlenstoffatomen steht, mit Ammoniak oder einem Alkylamin mit einem bis fünf Kohlenstoffatomen unter Erhalt eines Produkts, bei dem $R_1$ für Amino oder Alkylamino mit einem bis fünf Kohlenstoffatomen steht, 2) Reduktion einer Verbindung, bei der $R_2$ für $-CH_2-CH=CH-CH_2N_3$ steht, unter Erhalt eines Produkts, bei dem $R_2$ für $-(CH_2)_4NH_2$ steht, 3) selektive Entfernung der Schutzgruppen, und, falls erwünscht, Herstellung eines pharmazeutisch geeigneten Salzes des Produkts.

2. Verfahren nach Anspruch 1, wobei die Amid-Bindung zwischen den folgenden Fragmenten gebildet wird:

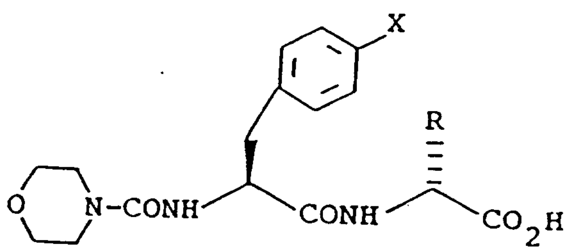

und

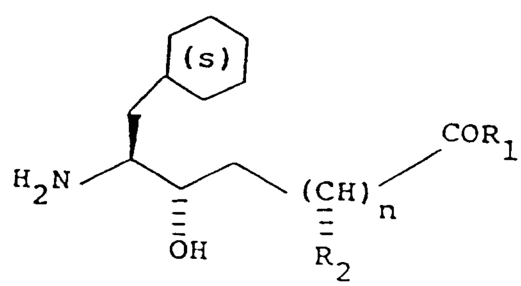

3. Verfahren nach Anspruch 1, wobei die Amid-Bindung zwischen den folgenden Fragmenten gebildet wird:

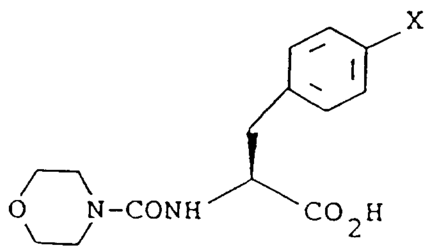

und

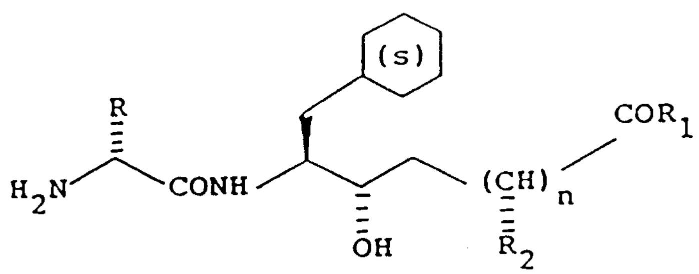

4. Verfahren nach Anspruch 1, wobei es sich bei dem Kupplungsreagenz um 1-Hydroxybenzotriazol und Dicyclohexylcarbodiimid handelt.

5. Verfahren zur Herstellung einer Verbindung mit der folgenden Formel:

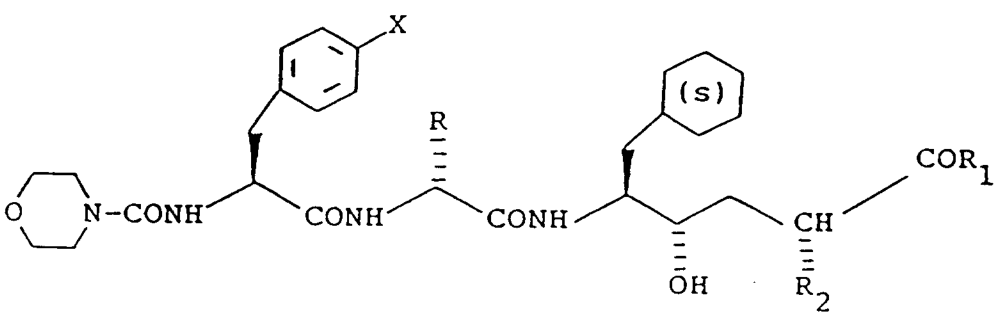

in der X für Wasserstoff, Methoxy oder Hydroxy steht, R für Alkyl mit einem bis sechs Kohlenstoffatomen, Imidazol-4-ylmethyl oder Methyldiomethyl steht, $R_1$ für Amino, Alkylamino mit einem bis fünf Kohlenstoffatomen, Alkoxy mit einem bis drei Kohlenstoffatomen oder 2-Alkoxycarbonylpyrrolidin-1-yl steht, wobei das Alkoxy ein bis drei Kohlenstoffatome hat, und $R_2$ für Alkyl mit drei oder vier Kohlenstoffatomen, $-CH_2C(Cl)=CH_2$, $-CH_2CH=CH-CH_2N_3$, - $(CH_2)_4NH_2$, $-CH_2C(Br)=CH_2$, $-CH_2CH=CHCl$, $-CH_2CH=C(CH_3)_2$, - $CH_2CH=CHCH_3$ oder $-CH_2C\equiv CH$ steht, dadurch gekennzeichnet, daß eine Verbindung mit der folgenden Formel:

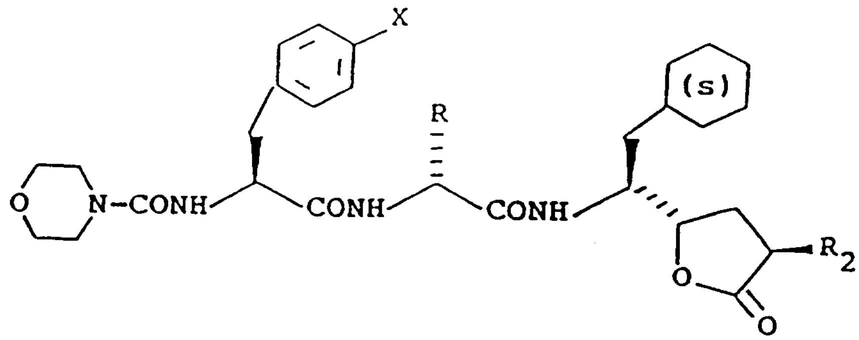

mit Ammoniak, einem Alkanol mit einem bis drei Kohlenstoffatomen, einem Alkylamin mit einem bis fünf Kohlenstoffatomen oder 2-Alkoxycarbonylpyrrolidin umgesetzt wird, wobei das Alkoxy ein bis drei Kohlenstoffatome hat, wobei die an der Umsetzung nicht beteiligten funktionellen Gruppen geschützt sein können, gefolgt von den folgenden wahlfreien Schritten, nämlich 1) Reduktion einer Verbindung, bei der $R_2$ für $-CH_2-CH=CH-CH_2N_3$ steht, unter Erhalt eines Produkts, bei dem $R_2$ für $-(CH_2)_4NH_2$ steht. 2) selektive Entfernung der Schutzgruppen und, falls erwünscht, Herstellung eines pharmazeutisch geeigneten Salzes des Produkts.

**Revendications**
**Revendications pour les Etats contractants suivants : AT, BE, CH, DE, FR, GB, IT, LI, LU, NL, SE**

**1.** Composé de formule

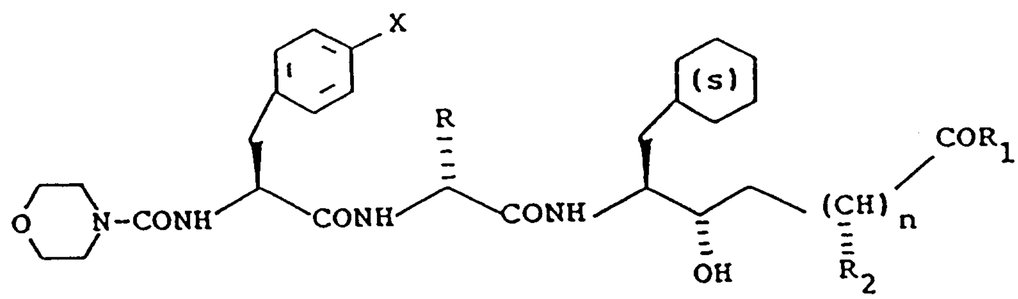

et un sel pharmaceutiquement acceptable de ce composé, formule dans laquelle X représente l'hydrogène, un groupe méthoxy ou hydroxy ; R est un groupe alkyle ayant 1 à 6 atomes de carbone, imidazole-4-ylméthyle ou méthylthiométhyle ; n est égal à 1, $R_1$ est un groupe amino, alkylamino ayant 1 à 5 atomes de carbone, alkoxy ayant 1 à 3 atomes de carbone ou 2-alkoxycarbonylpyrrolidine-1-yle, le groupe alkoxy ayant 1 à 3 atomes de carbone ; et $R_2$ est un groupe alkyle ayant trois ou quatre atomes de carbone, un groupe $-CH_2C(Cl)=CH_2$, $-CH_2CH=CH-CH_2N_3$, $-(CH_2)_4NH_2$, $-CH_2C(Br)=CH_2$, $-CH_2CH=CHCl$, $-CH_2CH=C(CH_3)_2$, $-CH_2CH=CHCH_3$ ou $-CH_2C{\equiv}CH$.

**2.** Composé suivant la revendication 1, dans lequel X est l'hydrogène et R est un groupe alkyle ayant trois ou quatre atomes de carbone.

**3.** Composé suivant la revendication 2, dans lequel R est un groupe n-butyle, $R_1$ est un groupe méthylamino et $R_2$ est un groupe isobutyle.

**4.** Composé suivant la revendication 2, dans lequel R est un groupe n-butyle, $R_1$ est un groupe méthylamino et $R_2$ est un groupe $-CH_2C(Cl)=CH_2$.

**5.** Composé suivant la revendication 1, dans lequel X est l'hydrogène et R est un groupe imidazole-4-ylméthyle.

**6.** Composé suivant la revendication 5, dans lequel $R_1$ est un groupe méthylamino et $R_2$ est un groupe isobutyle.

7. Composition pharmaceutique, comprenant une quantité à effet antihypertensif d'un composé suivant la revendication 1 et un diluant ou support acceptable du point de vue pharmaceutique.

**Revendications pour les Etats contractants suivants : ES, GR**

1. Procédé de production d'un composé de formule

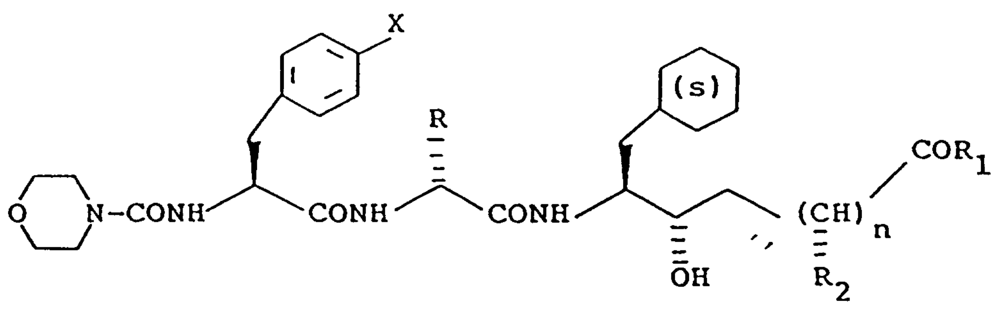

dans laquelle X représente l'hydrogène, un groupe méthoxy ou hydroxy ; R est un groupe alkyle ayant 1 à 6 atomes de carbone, imidazole-4-ylméthyle ou méthylthiométhyle ; n est égal à 1, $R_1$ est un groupe amino, alkylamino ayant 1 à 5 atomes de carbone, alkoxy ayant 1 à 3 atomes de carbone ou 2-alkoxycarbonylpyrrolidine-1-yle, le groupe alkoxy ayant 1 à 3 atomes de carbone ; et $R_2$ est un groupe alkyle ayant trois ou quatre atomes de carbone, un groupe $-CH_2C(Cl)=CH_2$, $-CH_2CH=CH-CH_2N_3$, $-(CH_2)_4NH_2$, $-CH_2C(Br)=CH_2$, $-CH_2CH=CHCl$, $-CH_2CH=C(CH_3)_2$, $-CH_2CH=CHCH_3$ ou $-CH_2C\equiv CH$, caractérisé par la formation d'une liaison amide, C(O)N, par couplage par déshydratation dans lequel les groupes fonctionnels non impliqués dans la réaction peuvent être protégés, suivie des étapes facultatives 1) de traitement d'un composé dans lequel $R_1$ est un groupe alkoxy de un à trois atomes de carbone avec l'ammoniac ou une alkylamine ayant un à cinq atomes de carbone pour obtenir le produit dans lequel $R_1$ est un groupe amino ou alkylamino ayant un à cinq atomes de carbone, 2) de réduction d'un composé dans lequel $R_2$ est un groupe $-CH_2-CH=CH-CH_2N_3$ pour obtenir le produit dans lequel $R_2$ est un groupe $-(CH_2)_4NH_2$, 3) d'élimination sélective des groupes protecteurs et, le cas échéant, de formation d'un sel pharmaceutiquement acceptable du produit.

2. Procédé suivant la revendication 1, dans lequel la liaison amide est formée entre les fragments

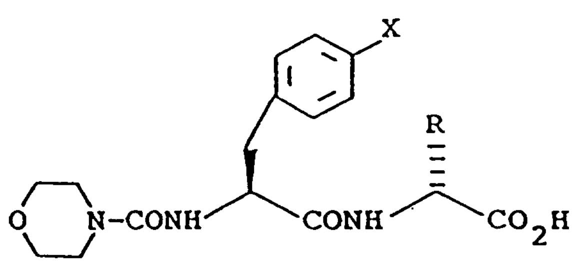

et

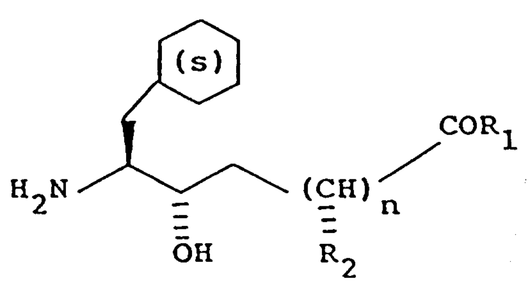

**3.** Procédé suivant la revendication 1, dans lequel la liaison amide est formée entre les fragments

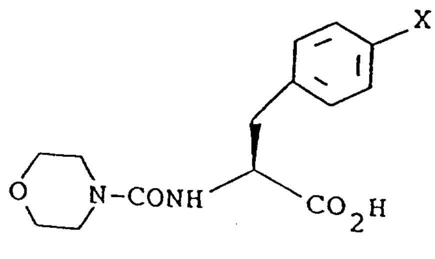

et

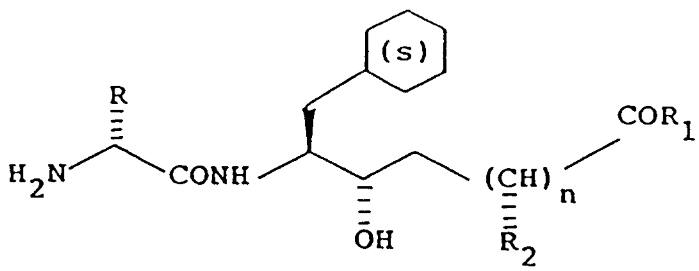

**4.** Procédé suivant la revendication 1, dans lequel le réactif de couplage est le 1-hydroxybenzotriazole et le dicyclohexylcarbodiimide.

**5.** Procédé de production d'un composé de formule

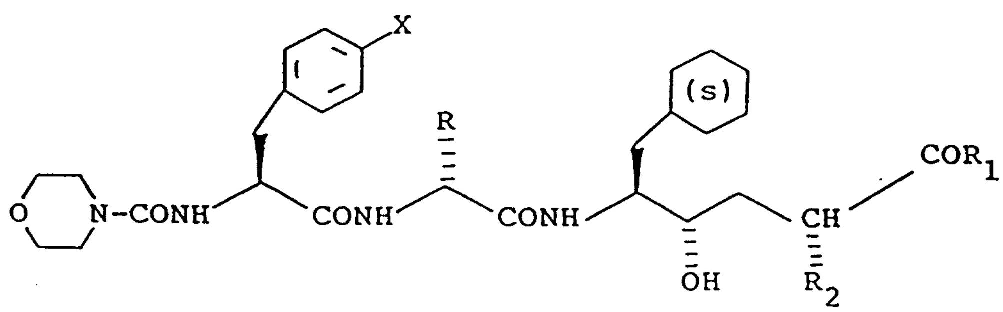

dans laquelle X représente l'hydrogène, un groupe méthoxy ou hydroxy ; R est un groupe alkyle ayant 1 à 6 atomes de carbone, imidazole-4-ylméthyle ou méthylthiométhyle ; $R_1$ est un groupe amino, alkylamino ayant un à cinq atomes de carbone, alkoxy ayant un à trois atomes de carbone ou 2-alkoxycarbonylpyrrolidine-1-yle, ledit groupe alkoxy ayant un à trois atomes de carbone ; et $R_2$ est un groupe alkyle ayant trois ou quatre atomes de carbone, $-CH_2C(Cl)=CH_2$, $-CH_2CH=CH\text{-}CH_2N_3$, $-(CH_2)_4NH_2$, $-CH_2C(Br)=CH_2$, $-CH_2CH=CHCl$, $-CH_2CH=C(CH_3)_2$, $-CH_2CH=CHCH_3$ ou $-CH_2C{\equiv}CH$, caractérisé par la réaction d'un composé de formule

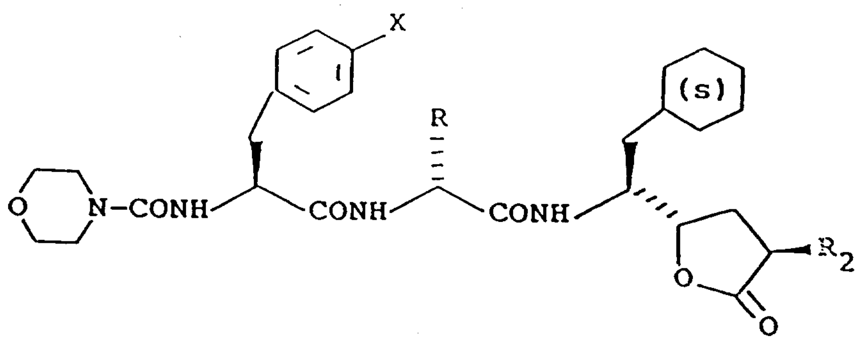

avec l'ammoniac, un alcanol ayant 1 à 3 atomes de carbone, une alkylamine ayant un à cinq atomes de carbone ou une 2-alkoxycarbonylpyrrolidine, le groupe alkoxy ayant un à trois atomes de carbone, où les groupes fonctionnels non impliqués dans la réaction peuvent être protégés, la réaction étant suivie des étapes facultatives 1) de réduction d'un composé dans lequel $R_2$ est un groupe $-CH_2-CH=CH-CH_2N_3$ pour former le produit dans lequel $R_2$ est le groupe $-(CH_2)_4NH_2$, 2) d'élimination sélective des groupes protecteurs et, le cas échéant, de formation d'un sel pharmaceutiquement acceptable du produit.